# EUROPEAN PATENT APPLICATION

(11) **EP 4 692 753 A1**
(43) Date of publication of application: **11.02.2026**
(21) Application number: 24193153.4
(22) Date of filing: 06.08.2024
(51) Int. Cl.: G01N 1/28, C12N 15/10, A61M 5/32, A61M 5/34, B01L 3/02, G01N 35/10

(54) **APPARATUS AND SYRINGE FOR FRAGMENTING NUCLEIC ACID SAMPLE MATERIAL**

(71) Applicant: Diagenode S.A., 4102 Seraing (Ougrée) (BE)
(72) Inventor: Lakhal, Wassim, 4102 Seraing (Ougrée) (BE)
(74) Representative: Maiwald GmbH

(57) **Abstract**

The present disclosure relates to syringe (1) for fragmenting nucleic acid sample material in liquid, the syringe (1) comprising a syringe body (2) extending at least in part in a longitudinal direction (L) of the syringe, a syringe plunger (4) moveable in the longitudinal direction (L) between a pulled-state, in which the plunger (4) is at least partially pulled out of the body (2) for receipt of liquid in the body (2), and a pushed-state in which the plunger (4) is at least partially received in the body (2) and liquid is pushed out of the body (2), and a cannula hub (6) comprising a cannula (7) for repeated withdrawal and discharge of liquid, based on repeated movement of the syringe plunger (4) between the pulled-state and the pushed-state, respectively, and an integrally or separately formed narrowing body (5) comprising a through hole (5a) extending in the longitudinal direction (L) and fluidly coupling the syringe body (2) and the cannula (7), and defining a cross-section of the flow of liquid at least in parts between the syringe body (2) and the cannula (7).

## Description

### TECHNICAL FIELD

The invention concerns an apparatus for fragmenting nucleic acid sample material, the use of said apparatus, a syringe for said apparatus, and the use of said syringe.

### BACKGROUND

As nucleic acids of interest may be too long for direct amplification or sequencing reactions such as long-read sequencing reactions, nucleic acid sample material may be fragmented prior to the amplification and/or sequencing reactions, e.g. the nucleic acids strands are broken up into smaller fragments. Nucleic acid fragment sizes for use in long-read sequencing reactions and similar reactions are typically 5 kb to 100 kb, more typically 15 to 40 kb. For at least some applications, however, a larger range of fragment sizes may be desirable for long-read sequencing.

For example, shearing forces may be applied to mechanically fragment DNA or RNA to a desired fragment size, such as by means of hydrodynamic shearing applications. Conventional devices for fragmenting DNA or RNA based on hydrodynamic shearing usually involve a filter screen with pores. Such devices, however, are also limited in the range of nucleic acid fragment sizes achievable, which is typically about 3 kb to about 70 kb for those devices.

### SUMMARY

Accordingly, there may be a need to make shearing more efficient, in particular as to the number of nucleic acid samples or amount of nucleic acid sample material that is being fragmented as well as to the range of nucleic acid fragment sizes. Also, it may be desirable to provide a reliable and reproducible outcome when shearing DNA or RNA sample material. Alternatively or additionally, improvements as to the sample throughput, fragment size range, accuracy, reproducibility and/or consistency of the shearing process may be desirable.

This is achieved by the subject matter of the independent claims, wherein further exemplary embodiments are included in the dependent claims and the following description.

A first aspect of the present disclosure relates to a syringe for fragmenting nucleic acid sample material in a liquid and/or fluid. The syringe comprises a syringe body extending at least in part in a longitudinal direction of the syringe, and a syringe plunger moveable in the longitudinal direction between a pulled-state, in which the plunger is at least partially pulled out of the body for receipt of liquid in the body, and a pushed-state in which the plunger is at least partially received in the body and liquid is pushed out of the body. The syringe further comprises a cannula hub (also referred to herein as "hub" or "needle hub") comprising a cannula for repeated withdrawal and discharge of liquid, based on repeated movement of the syringe plunger between the pulled-state and the pushed-state, respectively. The syringe further comprises a narrowing body comprising a through hole extending in the longitudinal direction and fluidly coupling the syringe body and the cannula. The narrowing body and/or the through hole thereof defines and/or narrows a cross-section of the flow (also referred to herein as flow cross-section) of liquid at least in parts between the syringe body and the cannula. Therein, the narrowing body may be integrally formed with the cannula hub or may be formed as separate part, as described further below.

The syringe of the present disclosure may support improved, more efficient, and more consistent fragmentation of nucleic acid sample material. For example, by providing a narrowing body comprising a through hole in the longitudinal direction, a fluid flow in the longitudinal direction may be improved, in particular such that the shearing outcome can be improved, for example in terms of one or more of accuracy, reproducibility and consistency.

In particular, a broader range of nucleic acid fragment sizes can be achieved with the syringe according to the present disclosure, optionally within shorter period of time or with fewer repetitions of piston movement, when compared to conventional syringes usable for shearing. For instance, the syringe according to the present disclosure may allow for a nucleic acid fragment size range of about 2 kb to about 150 kb, for example from about 10 kb to about 150 kb.

The syringe of the disclosure may be used in an apparatus of the present disclosure. An apparatus of the present disclosure may optionally be configured to receive a plurality of syringes of the disclosure.

For the present disclosure, the longitudinal direction may be seen as running along the longitudinal axis and/or along length direction of a syringe.

The "nucleic acid fragment range size" achievable with the syringe and/or apparatus of the present disclosure may refer to an overall range of the mean or average lengths of nucleic acid fragments that can be obtained based varying one or more parameters or protocols for the shearing, such as e.g. a number of repetitions of the piston movement, cannula size, sample volume, concentration of nucleic acid in the sample volume, motor speed, or the like.

It is noted that in a single shearing process, for example applying a particular set of parameters or a particular protocol, a distribution of fragment lengths centered around a mean or average fragment length may be obtained, because mechanical shearing produces a Gaussian-like distribution of the fragment length rather than a sharp peak. In the context of the present disclosure, the "accuracy of the shearing process" relates to such distribution of fragment lengths obtainable in a single shearing process, for example applying a particular set of parameters or a particular protocol, such as e.g. a number of repetitions of the piston movement, cannula size, sample volume, concentration of nucleic acid in the sample volume, motor speed, or the like. Accordingly, the term "accuracy of the shearing process", may relate to the range or distribution of fragment lengths (or sizes) of sheared DNA fragments with respect to the expected or desired fragment size (e.g. based on validation experiments), when specific parameters or a specific protocol is applied. The mean or peak fragment length or fragment size of such distribution may be referred to as the target fragment size or length, which can be considered as the average size or length of the whole curve or distribution. For instance, a more accurate shearing process may mean that a narrower distribution of fragment lengths may be obtained, for example which may be sharply centered around the target fragment size.

Further, in the context of the present disclosure, the "reproducibility of the shearing process" may relate to the variation in fragment length distributions between multiple samples sheared in the same run, for example using multiple syringes in a multi-well plate as described herein. For a reproducible shearing process, the coefficient of variation (CV%) in the mean or average fragment length of the fragment length distributions between multiple samples sheared in the same run should be below about 30%, preferably below about 20%.

Moreover, consistency of the shearing process may relate to the variation in fragment length distribution over one or more of time, devices, apparatuses, syringes, batches of devices or syringes, or users. Accordingly, a consistent shearing process may mean that variations (e.g. the CV%) in the mean or average fragment length of the fragment length distributions over one or more of time, devices, apparatuses different syringes, batches of devices or syringes, or users are below 30%, preferably below 20%.

It is noted that one or more features of the syringe and/or the apparatus described herein synergistically interact to provide for an improvement of one or more of the sample throughput, fragment size range, accuracy, reproducibility and/or consistency of the shearing process. In other words, one or more features of the syringe and/or the apparatus described herein contribute to improving one or more of the sample throughput, fragment size range, accuracy, reproducibility and/or consistency of the shearing process.

In particular, by means of the syringe with the narrowing body that comprises the through hole defining the flow cross-section body, the amount of air inside the cannula hub may be reduced or minimized, which can reduce the risk of air bubbles to potentially interfere with the liquid flow, and e.g. increase the variability of the shearing outcome, for example between different syringes or between consecutive shearing processes or runs. Hence, by means of the syringe with narrowing body an improvement to one or more of accuracy, reproducibility and/or consistency of the shearing can be provided.

The narrowing body may generally be configured and/or arranged to fill a dead space or dead volume within the syringe and/or cannula hub. Such dead space may be defined by one or more interior walls of the cannula hub, as illustrated and discussed in further detail herein below. Accordingly, the narrowing body may be regarded as or referred to herein as a filling body or filling element configured to fill or reduce at least a part of the dead space in the cannula hub.

As used herein, the term "flow cross-section" or "cross section of the flow" may refer to the cross-sectional area of the flow path through the narrowing body and/or the through hole thereof, through which the liquid may flow when passing through the narrowing body. In particular, the flow cross-section of the narrowing body may refer to or denote the cross-sectional area of the through hole of the narrowing body, which through hole defines or provides a passage or flow path for the liquid. In this context, the phrase "narrowing the cross-section of the flow" or "narrowing the flow cross-section" may refer to a narrowing resulting from or caused by the narrowing body compared to a conventional syringe without narrowing body, respectively, a syringe where the dead space in the cannula hub is not filled. Also, since the through hole of the narrowing body defines the cross-section of the flow path through the narrowing body, the phrases narrowing the flow cross section" and "defining the flow cross section" may be interchangeably used herein.

The narrowing body may have a single through hole. Optionally, the cross-section of the through hole and/or flow cross section may be substantially constant along the length of the through hole in longitudinal direction of the syringe. Therein, substantially constant may mean that variations in the cross-sectional diameter (also referred to herein a through hole diameter) or cross-sectional area of the through hole are below about 20%, preferably below about 10%.

For example, the cross section or flow cross section of the through hole and/or the flow cross section may be constant over at least 80%, preferably over at least 85%, of an entire length or extension of the through hole or narrowing body measured along the longitudinal direction. As used herein, a constant cross section or constant flow cross section of the through hole can relate or refer to a constant area and/or diameter of the cross-section of the through hole along at least a part of the length of the through hole in longitudinal direction.

Accordingly, a predetermined or constant cross section of the through hole and/or a predetermined or constant flow cross section may be provided by the narrowing body, which can allow to provide for an accurate, reproducible and/or consistent shearing. The cross-sectional area of the through hole may have a round, rounded, rectangular, elliptic, oval, or polygon-like shape.

The syringe may be a consumable, e.g. the syringe can be disposable, preferably but not necessarily after a single use.

The syringe is configured for repeatedly flowing liquid through the syringe, the liquid optionally including nucleic acid sample material. The syringe may be a dedicated shearing syringe. Alternatively, the syringe is a commercial syringe adapted for shearing nucleic acid sample material.

The syringe body and the cannula of the syringe are in liquid communication. The syringe body and the cannula hub may be integral, e.g. a single piece.

The cannula may be configured as a needle, e.g. have a sharp distal tip, but may alternatively have a blunt distal termination.

In some embodiments, the narrowing body may be an integral part of the cannula hub, and optionally of the syringe. For example, the syringe may comprise a cannula hub with the narrowing body integrated into the cannula hub. In some embodiments, the syringe body, the cannula hub and the narrowing body are altogether integrally formed, e.g. one single piece. In this case, the syringe body and/or the hub and the narrowing body may optionally be made of e.g. a solid material, as described below for the narrowing body.

In some embodiments, the narrowing body may be a separate insert piece or part that is placed in the cannula hub, and optionally the syringe. For example, the syringe may comprise a cannula hub and a separate insert component that is inserted into the body of the cannula hub, e.g. defined by one or more interior walls of the cannula hub, such that the dead space within the cannula hub is at least partially filled by the insert. A narrowing body that is a separate insert can for example used to adapt common commercial syringes for shearing applications, though the insert is not limited to use with commercial syringes.

In some embodiments, the syringe body, the cannula hub and the narrowing body are altogether integrally formed, e.g. one single piece or part. Optionally, one of, a plurality of or each of the syringe body, the cannula hub and the narrowing body can be made of e.g. a solid material, as described below for the narrowing body in more detail.

Optionally, the syringe includes an insert as the narrowing body. In other words, the narrowing body and the cannula hub may be formed as separate parts, wherein the narrowing body may be insertable (as an insert) into the cannula hub. The insert as narrowing body, or vice versa, the narrowing body formed as insert, may be insertable into the cannula hub, such that a dead space within the cannula hub is at least partly filled by the narrowing body,. Hence, the narrowing body may be embodied, shaped or formed as an insert. Providing a separate narrowing body to be inserted into the cannula hub, e.g. the syringe including the cannula hub, may allow for adaption of a common syringe for nucleic acid shearing.

Optionally, the cannula hub protrudes from the syringe body by about 5 mm to 10 mm, optionally about 7 mm to 9 mm in the longitudinal direction.

Further optionally, the needle or cannula may be positioned inside a single well to allow the treatment or processing of the majority of the sample volume contained in the well, for example more than about 70%, preferably more than about 80%, and more preferably more than about 85% of the sample volume in the well. For example, the tip of cannula or needle may be placed about 0.2 to 5 mm from the well bottom, more optionally about 1 mm. Accordingly, the tip of the cannula may be positioned within the lower half, preferably within the lower quarter of the volume of the well.

To support a sufficiently strong Luer connection between the cannula hub and the syringe body/tip, a height of narrowing body (e.g. formed as insert or as separate part), which may be referred to as length of the narrowing body in the longitudinal direction, may optionally be between 2.5 mm to 2.8 mm, for example about 2.65 mm. The remaining space in the cannula hub, e.g. above the narrowing body may be available for engagement with the syringe body or the syringe tip, e.g. for a sufficiently strong Luer connection.

In an example, the length or height of the narrowing body may be about at least one fourth of the length of the cannula hub (also referred to herein as "hub") in the longitudinal direction. Additionally or alternatively, the length or height of the narrowing body may be between about 25 and 35%, optionally between about 27 and 33%, for example between about 28.7 % and 32.2 %, in particular about 30.5% of the length of the cannula hub. A cannula may have a size of 0.5 to 1.5 inch, preferably 0.5 inch. A suitable cannula size is e.g. 27 G and 0.5 inch.

Optionally, the through hole has a through-hole diameter and the cannula has an inner cannula diameter. Exemplary sizes are for the inner cannula diameter: 0.1 - 0.8 mm; and for the through hole diameter of the narrowing body: 0.5 to 1.5 mm.

The through-hole diameter may be larger than the inner cannula diameter and/or the through-hole diameter is about 1.5 to 20 times, more optionally 1.5 to 10 times, still more optionally 2 to 6 times, the inner cannula diameter. These dimensions may help to improve the shearing output.

Optionally, the cannula protrudes at least 3 mm, optionally at least 5 mm from the cannula hub, and/or less than 38, optionally less than 26 mm, more optionally less than 13 mm, from the cannula hub. More optionally, a preferable range may be 3 to 26 mm. This may help to improve shearing output.

Optionally, the cannula is free of a filter defining multiple through-passages. More optionally, the cannula may be free of a hydropore. However, it is not excluded that the cannula additionally comprises a hydropore. In other words, a filter may further support the fragmentation process.

Optionally, the narrowing body (e.g. formed as insert or as separate part) is at least in part or in its entirety made of a solid material, such as a bioplastic and/or a thermoplastic polymer, such as PLA (Polylactic acid, also known as poly(lactic acid) or polylactide (PLA), e.g. for 3D printing of the narrowing body) and/or PP (Polypropylene, e.g. for injection molding of the narrowing body).

An advantage of solid materials relative to elastic, resilient materials for the narrowing body may be that the through hole, in particular the cross-section, cross-sectional area and/or diameter thereof, may be well defined and may be substantially constant along an extension or length of the through hole in longitudinal direction. This may mean that the diameter of the through hole for flowing the fluid or liquid, and hence the flow cross-section, can be predetermined and/or fixed, which can allow for a well-defined fluid flow rate, e.g. a better controlled flow. Also, accuracy, reproducibility and/or consistency of the shearing process, for example when using multiple syringes and/or across consecutive shearing processes, can be improved due to the fact that the narrowing body can minimize the amount of the air inside the cannula hub and thus reducing the risk of air bubbles to potentially interfere with the liquid flow increasing randomly the variability of the shearing outcome between different syringes or between consecutive shearing processes. Also, using solid material for the narrowing body may allow to flow the liquid at high pressure and/or flow rate therethrough, for example without substantially expanding the through-hole, which could be the case when using elastic material. Accordingly, uncontrolled or uncontrollable variations in the diameter and/or area of the cross-section of the through hole can be advantageously reduced or avoided when using solid material for the narrowing body.

In conventional syringes, inserts may usually be used to fill certain parts of the syringe in order to reduce the amount of lost liquid, for example a medicament applied via the syringe. Such conventional inserts are made of elastic material, because elastic material can be compressed to provide a sealing effect and hence reduce leakage or spillage of the liquid.

The inventors of the present invention, however, surprisingly found that using solid material for the narrowing body is of particular advantage for shearing DNA or RNA sample material. Specifically, it has been surprisingly found that the overall range of nucleic acid fragment sizes, as well as the accuracy, reproducibility and/or consistency of the outcome of the shearing process can be significantly improved when using solid material for the narrowing body.

Exemplary and non-limiting physical properties of suitable materials for a solid narrowing body may include one or more of the following:
- Density 1.24 g/cm³;
- Vicat Softening Temperature 57 °C;
- Heat Deflection Temperature 57 °C;
- Melting Temperature 160 °C;
- Melt Index 42.4 ± 3.5 g/10 min;
- Tensile Strength 35 ± 4 MPa;
- Breaking Elongation Rate 12.2 ± 1.8 %;
- Bending Modulus 2750 ± 160 MPa;
- Bending Strength 76 ± 5 MPa; and
- Impact Strength 26.6 ± 2.8 kJ/m².

However, narrowing bodies made of an elastic material, e.g. made of an elastomer, e.g. a resin, are not excluded. In this case, SLA resin (e.g. resin manufactured including stereolithography) may be used. The resin may be a photopolymer or light-activated resin. Photosensitive monomers and oligomers, combined in short chains, which turn into long chains with post-curing, may be used.

Exemplary and non-limiting physical properties of suitable materials for an elastic narrowing body may include one or more of the following:
- Tensile strength 32 - 60 MPa;
- Tensile modulus 2500 - 2800 MPa;
- Tensile elongation 7 - 9%;
- Flexural strength 51 - 93 MPa;
- Flexural modulus 2200 - 2775 MPa;
- HDT (0.45 MPa) 48 - 70°C; and
- HDT (1.8 MPa) 120 - 130°C.

The narrowing body, more specifically, its outer shape, may at least in part be cone-shaped. Accordingly, at least a part of the narrowing body may be cone-like shaped or formed.

The narrowing body may be injection molded or 3D printed. Such narrowing body may be beneficial in terms of sustainability and/or manufacture.

Optionally, the syringe further comprises a syringe tip at a distal end of the syringe body, wherein the syringe tip and the cannula hub are configured such that they are connectable and/or connected to each other via a Luer connection or a luer-lock connection. A Luer connection may be seen as relying on a friction fit between syringe body and cannula hub, such as a luer-slip or slip-tip. A luer-lock connection may be regarded as relying on a threaded locking connection. These connections may provide for a reliable and swift connection between the syringe tip and the cannula hub.

Optionally, a distal end of the cannula hub is tapered, more optionally configured to engage a liquid reservoir and/or configured to be received by a holder. Further optionally, the distal end of the cannula hub may have a specific/dedicated shape of the outer side of distal end of the cannula hub, in particular for fitting into a holder/grouping mount. This may be beneficial for the connection between the cannula hub, e.g. syringe, and the reservoir and/or the holder.

Optionally, a cannula size range is from 18 G to 34 G, more optionally from 20 to 32 G, still more optionally from 25 G to 30 G, even more optionally the cannula size is 27 G. This may improve the shearing output.

Optionally, the syringe body comprises body engagement means for engagement with a retainer. More optionally, the body engagement means is arranged at a proximal end of the syringe body and/or protrudes transverse to the longitudinal direction from the syringe body from the remaining (cylindrical) syringe body. For example, the body engagement means may protrude between about 0.5 mm to about 8 mm, for example between about 1 mm and about 6 mm from the syringe body. This may help to provide for a reliable connection/engagement between the syringe and the apparatus, more specifically with respect to the syringe body and the retainer.

The body engagement means may be configured for engagement with the retaining means for holding the syringe body in place. Additionally or alternatively, the engagement means may be embodied as a circumferential protrusion. In some embodiments, the outer diameter may be less than 8 mm, optionally 7 mm, more optionally 6 mm. This may allow for simple and efficient realization of engagement means. Also, this may help to position the syringes next to each other in e.g. a well plate having a predefined pitch, determining a spacing between adjacent syringes. The body engagement means may generally allow for a tight mechanical connection or assembly with the retainer of the apparatus, which can allow to provide an accurate, reproducible and/or consistent shearing outcome, respectively, outcome of the shearing process.

Optionally, the plunger comprises plunger engagement means for engagement with an actuator. More optionally, the plunger engagement means is arranged at a proximal end of the plunger and/or protrudes transverse to the longitudinal direction from the plunger, optionally less than 7 mm, more optionally 5 mm, from the remaining (cylindrical) syringe body. This may help to allow for a reliable connection/engagement between the syringe and the apparatus, more specifically with respect to the plunger and the actuator. In some embodiments, this may support initiating reciprocating movement by means of the actuator. Also, the plunger engagement means may generally allow for a tight mechanical connection or assembly with the actuator of the apparatus, which can allow to provide an accurate, reproducible and/or consistent shearing outcome.

Additionally or alternatively, the plunger engagement means may be embodied as a circumferential protrusion. In some embodiments, the outer diameter may be less than 7 mm, optionally 6 mm, more optionally 5 mm. This may allow for simple and efficient realization of plunger engagement means. Also, this may help to position the syringes next to each other in e.g. a well plate having a predefined pitch, determining a spacing between adjacent syringes.

Optionally, the cannula hub comprises a proximal (de)limiter, more optionally a circumferential ring, for limiting insertion of the syringe in the longitudinal direction into a holder and/or a reservoir. More optionally, the limiter is a stop of the cannula hub for limiting an insertion depth in the grouping mount and, thus, into the reservoir/well plate. This may support a well-defined and reliable abutment/connection between the syringe and the holder and/or reservoir.

A second aspect of the present disclosure relates to the use of the syringe of the disclosure for repeatedly flowing liquid through the syringe, wherein the liquid contains nucleic acid sample material and is, via the cannula, withdrawn from a liquid reservoir (e.g. reservoir for receiving liquid) and discharged into the reservoir multiple times, such that the nucleic acid sample material is sheared and fragmented.

A third aspect of the present disclosure relates to an apparatus for fragmenting nucleic acid sample material in liquid by means of a plurality of syringes, each syringe having a syringe body and a syringe plunger moveable relative to the syringe body. The apparatus comprises a support for directly or indirectly supporting a plurality of reservoirs for receipt of liquid, and at least one grouping mount for positioning each of the plurality of syringes above the respective reservoir for fluid coupling to the respective reservoir. The plurality of reservoirs and the plurality of syringes are each arranged in a 2D matrix arrangement. The apparatus further comprises at least one retainer configured to retain one of the plurality of syringe bodies and the plurality of plungers in the 2D matrix arrangement stationary relative to the support, and at least one actuator is configured to reciprocate the other one of the plurality of syringe bodies and the plurality of plungers in the 2D matrix arrangement along a longitudinal direction, in which the respective body of each of the plurality of syringes extends.

A fourth aspect of the present disclosure relates to an apparatus for fragmenting a nucleic acid sample in liquid by means of a plurality of syringes, each syringe comprising a cannula, a syringe body and a syringe plunger moveable relative to the syringe body. The apparatus comprises a support for receiving a plurality of reservoirs arranged in a predetermined pitch, the support configured as a common support for the plurality of reservoirs, and further comprises a removable grouping mount comprising a plurality of mounting means arranged in the predetermined pitch, the grouping mount configured as a common mount for the plurality of syringes in the predetermined pitch, wherein each mounting means is configured to capture a lower part of the respective syringe and has a through-hole for passing through of at least a respective cannula, the cannula protruding downwards from the lower part of the syringe. The apparatus further comprises at least one retainer configured to retain one of the plurality of syringe bodies and the plurality of plungers in the predetermined pitch stationary relative to the support, and at least one actuator configured to reciprocate the other one of the plurality of syringe bodies and the plurality of plungers in predetermined pitch along a longitudinal direction, in which the respective body of each of the plurality of syringes extends.

The present disclosure is directed to embodiments offering improved and more efficient fragmentation of nucleic acid (e.g. RNA and/or DNA, in particular DNA) sample material, specifically regarding the accuracy, reproducibility and/or consistency of the shearing outcome. Also, by arranging the reservoirs and/or syringes in a 2D matrix arrangement, space may more efficiently be used for shearing nucleic acid by means of syringes, wherein the syringes are located at predetermined positions. Additionally or alternatively, a grouping mount representing a common mount for a plurality of syringes in a predetermined pitch can be provided, wherein the mount may have mounting means having a through-hole for the cannula of the syringe, which may allow for more efficient and reliable positioning of syringes in the apparatus. The apparatus may allow for a range of nucleic acid fragment size of about 2 kb to about 150 kb, for example about 10 kb to about 150 kb.

The apparatus of the disclosure may be referred to as a shearing apparatus.

For the present disclosure, the plurality of reservoirs may be embodied as a well plate, e.g. a standard 96 well plate. The plurality of reservoirs may non-fluidly be connected/attached to each other. Each syringe, e.g. each shearing entity, is associated with a respective/corresponding reservoir, which is not in fluid communication with the remaining reservoirs.

The apparatus may be configured for receipt of syringes arranged in multi-well plate, e.g. the pitch of a 96-well PCR reaction plate.

The plurality of reservoirs may form a single reservoir plane, e.g. have a common plane (e.g. upper and/or lower end) of the reservoirs, meaning that the reservoirs are on or are arranged in the same plane. The same horizontal level may be provided for all syringes and/or reservoirs.

The two-dimensional matrix arrangement of the reservoirs and syringes may be regarded as a grouping of reservoirs and syringes extending in first and second directions. The syringes may be positioned next to each other in depth direction (x), in the horizontal plane (xy). The grouping may be in a plane, namely the xy plane.

The retainer may have a plurality of retainer elements, e.g. one retainer element per syringe. By means of the retainer and/or retainer elements a tight mechanical connection with the syringes can be ensured, which can allow to provide an accurate, reproducible and/or consistent shearing outcome, respectively, outcome of the shearing process.

The actuator may be moved by one or more motors up and down, in particular parallel to the longitudinal direction, to move the plungers between a pulled and pushed state, e.g.to move the plungers back and forth. Hence, the actuator may be configured for reciprocating movement in the longitudinal direction (L). The actuator may have a plurality of actuator elements, e.g. one actuator element per syringe.

The retainer and/or actuator may be "filled" in the depth direction (x) with syringes. Also, multiple syringes are positioned side-to-side (in the y-direction).

The apparatus may be seen as defining a cavity for housing/receiving the plurality of syringes.

The apparatus may be configured to receive syringes standing upright on the support. Each of the plurality of syringe bodies may extend in the longitudinal direction, when received in the apparatus.

The apparatus may comprise an, optionally removable, holder. The holder and the retainer may be integral (one piece), e.g. integrally formed. The holder may be configured for holding a (96) well plate.

The holder/grouping mount may be embodied as a plate and/or may be referred to as a shearing plate. A thickness of the grouping mount may be from 1 mm to 20 mm, e.g. such that a cannula tip of a syringe extends into the reservoir, optionally hits the bottom of reservoir. By means of the holder and/or grouping mount, a tight mechanical connection with the syringes can be ensured, which can allow to provide an accurate, reproducible and/or consistent shearing outcome, respectively, outcome of the shearing process.

The apparatus may be configured for fluid coupling of a cannula to the respective reservoir by the cannula protruding into reservoir, optionally the cannula reaching the reservoir bottom.

The apparatus of the third and fourth aspects are compatible, so that an apparatus of the disclosure may realize at the same time the characteristics of the third and fourth aspects.

Specifically, optionally, in the apparatus of the third aspect, the support may further be configured for receiving the plurality of reservoirs arranged in a predetermined pitch, the support configured as a common support for the plurality of reservoirs; the apparatus may further comprise the removable grouping mount comprising a plurality of mounting means arranged in the predetermined pitch, the grouping mount configured as a common mount for the plurality of syringes in the predetermined pitch, each mounting means configured to capture a lower part of the respective syringe and having a through-hole for passing through of at least a respective cannula, the cannula protruding downwards from the lower part of the syringe.

Optionally, in the apparatus of the fourth aspect, the support is further configured for directly or indirectly supporting the plurality of reservoirs for receipt of liquid, and the grouping mount for positioning each of the plurality of syringes above the respective reservoir for fluid coupling to the respective reservoir; wherein the plurality of reservoirs and the plurality of syringes are each arranged in a 2D matrix arrangement, the at least one retainer configured to retain one of the plurality of syringe bodies and the plurality of plungers in the 2D matrix arrangement stationary relative to the support, and the at least one actuator configured to reciprocate the other one of the plurality of syringe bodies and the plurality of plungers in the 2D matrix arrangement along a longitudinal direction, in which the respective body of each of the plurality of syringes extends.

Optionally, the grouping mount is located above the support and configured such that, when the plurality of reservoirs is received by the support and the plurality of syringes is captured by the grouping mount, each cannula extends into the respective reservoir to establish fluid communication with liquid contained in the respective reservoir.

Optionally, the apparatus is configured to repeatedly switch between a pulled-state of the respective plunger of each syringe in which the respective plunger is at least partially pulled out of the body for receipt of liquid in the body, and a pushed-state of the respective plunger of each syringe in which the respective plunger is at least partially received in the respective body and liquid is pushed out of the body, in the 2D matrix arrangement for repeated withdrawal and discharge, respectively, of liquid by means of the reciprocating movement of the actuator along the longitudinal direction.

Optionally, the 2D matrix arrangement comprises rows running in first (x) and second (y) directions, each of the first and second directions angled, optionally perpendicular, to the longitudinal direction, wherein the at least one retainer has a plurality of retainer elements that are adjacent to each other in the second direction (y) and accessible by the syringes in first direction (x). Each retaining element may be configured to retain, among the plurality of syringes, a subgroup of syringes aligned in the row running in the first direction (x), and/or the at least one actuator has a plurality of actuator elements that are adjacent to each other in the second direction (y) and accessible by the syringes in the first direction (x), and each actuator element is configured to retain, among the plurality of syringes, a subgroup of syringes aligned in the row running in the first direction (x).

A row in the first direction, e.g. in the x-direction, is characterized by same position in the second direction, e.g. the y-direction.

Optionally, the support includes a drawer moveable in a transverse direction (x, y) orthogonal to the longitudinal direction.

Optionally, the apparatus comprises a magnetic connector at the holder for detachably attaching the at least one liquid reservoir, optionally at the plurality of reservoirs, more optionally at the well plate, to the holder. More optionally, a reservoir or a PCR plate may also comprise a counterpart connector.

Optionally, the apparatus further comprises a door for access, in particular to the support, optionally the door having reinforcement means for reinforcement of the at least one retainer, when the door is in a closed state. More optionally, a crossbeam or arm in the door closes the retainer elements from the opposite side. This may support the fixation and reliable positioning of the retainer.

Optionally, the apparatus further comprises at least one pillar extending in the longitudinal direction, more optionally two pillars running in parallel in the longitudinal direction, and supporting the holder and/or the support, optionally at opposite sides in the transverse direction (xy). This may provide for a stable structure of the apparatus.

Optionally, the apparatus further comprises at least one motor, more optionally configured to run at different speeds, more optionally two motors and/or located at a lower part of the apparatus. The motor(s) are configured to move the actuator in the longitudinal direction. This may allow for reliable reciprocation of the plungers.

Optionally, a stepper motor is used. The stepper motor may allow for adjustment of RPM to accommodate for different DNA/RNA fragmenting needs. Solely by way of example and not limitation, a stepper motor may be configured to be adjusted from about two thousand (2.000) RPM to about ten thousand (10.000) RPM. A speed of the motor may, alternatively or additionally, be characterized by 0 and one thousand (1.000) steps per second. Optionally, motors may have synchronized motor controls. This may support reliable reciprocation of the plungers.

Optionally, the retainer is configured to restrict movement in the longitudinal direction of the plurality of syringe bodies, more optionally when aligned in the transverse direction (x, y), by abutment of body engagement means of each syringe body against the retainer. This may allow for reliable fixation of the syringes. More optionally, the body end may protrude in the transverse direction from the body of the syringe. Alternatively or additionally, multiple transverse rows of retainers may be provided in the apparatus. The transverse direction may e.g. be in the x-y plane in the attached drawings.

The retainer(s) may align/fix one or more syringes relative to each other. Alternatively or additionally, the retainer(s) may restrict movement of the plurality of plungers in the longitudinal direction.

Optionally, the actuator is configured to engage with a plunger end of the plunger of each of the plurality of syringes, optionally when aligned in the transverse direction (x, y), by capturing plunger engagement means of each of the plungers. More optionally, the plunger end may protrude in the transverse direction from the plunger. The apparatus may comprise multiple transverse lines of actuators. This may represent an efficient way of implementation.

The apparatus may be configured for reciprocating movement of the plurality of plungers. Alternatively, apparatus may be configured for reciprocating movement of the plurality of syringe bodies.

Optionally, the retainer is located below the actuator, seen in the longitudinal direction, and/or the retainer and the actuator each extend at least in a transverse direction (x, y) perpendicular to the longitudinal direction.

A fifth aspect of the present disclosure relates to a use of the apparatus of the disclosure. The use comprises arranging the plurality of reservoirs at the grouping mount, and arranging the grouping mount comprising the plurality of syringes, while each syringe is captured by the respective grouping means, in the apparatus. The grouping mount is located above the support and each cannula extends into the respective reservoir. The use further comprises establishing fluid communication, via the respective cannula, between each syringe and the liquid contained in the respective reservoir.

The invention is defined in the claims. However, below there is provided a non-exhaustive list of non-limiting exemplary embodiments. Any one or more of the features of these exemplary embodiments may be combined with any one or more features of another example, embodiment, or aspect described herein.

Embodiment one is a syringe for fragmenting nucleic acid sample material in liquid, the syringe comprising: a syringe body extending at least in part in a longitudinal direction (L) of the syringe, a syringe plunger moveable in the longitudinal direction (L) between a pulled-state, in which the plunger is at least partially pulled out of the body for receipt of liquid in the body , and a pushed-state in which the plunger is at least partially received in the body and liquid is pushed out of the body , and a cannula hub comprising a cannula for repeated withdrawal and discharge of liquid, based on repeated movement of the syringe plunger between the pulled-state and the pushed-state, respectively, and an integrally or separately formed narrowing body comprising a through hole extending in the longitudinal direction (L) and fluidly coupling the syringe body and the cannula , and defining a cross-section of the flow of liquid at least in parts between the syringe body and the cannula .

Embodiment two is the syringe of embodiment one, wherein the syringe includes an insert as narrowing body insertable into the cannula hub such that a dead space within the cannula hub is at least partly filled by the insert, the dead space defined by one or more interior walls of the cannula hub .

Embodiment three is the syringe of embodiments one or two, wherein the through hole has a through-hole diameter and the cannula has an inner cannula diameter, wherein the through-hole diameter is larger than the inner cannula diameter and/or the through-hole diameter is about 1.5 to 20, more optionally 1.5 to 10, still more optionally 2 to 6, times the inner cannula diameter.

Embodiment four is the syringe of any of embodiments one to three, wherein the cannula protrudes at least 3 mm, optionally at least 5 mm from the cannula hub, and/or less than 38, optionally less 26 mm, more optionally less than 13 mm, from the cannula hub .

Embodiment five is the syringe of any of embodiments one to four, wherein the cannula is free of a filter defining multiple through-passages.

Embodiment six is the syringe of any of embodiments one to five, wherein the narrowing body is at least in part made of a solid material, such as a bioplastic, such as Polylactic acid, and/or is at least in part cone-shaped and/or is 3D printed.

Embodiment seven is the syringe of any of embodiments one to six, wherein the syringe further comprises a syringe tip at a distal end of the syringe body , wherein the syringe tip and the cannula hub are configured such that they are connectable and/or connected to each other via a Luer connection or a luer-lock connection, and/or a length (l) of the narrowing body in the longitudinal direction (L) is about between 25 and 35%, more optionally between 27 and 33% of the length of the cannula hub in the longitudinal direction (L).

Embodiment eight is the syringe of any of embodiments one to seven, wherein a distal end of the cannula hub is tapered, optionally configured to engage a liquid reservoir and/or configured to be received by a grouping mount.

Embodiment nine is the syringe of any of any of embodiments one to eight, wherein a cannula size is between 18 to 34 G, optionally 20 and 32 G, more optionally 25 to 30 G, still more optionally at 27 G.

Embodiment ten is the syringe of any of any of embodiments one to nine, wherein the syringe body comprises body engagement means for engagement with a retainer, preferably wherein the body engagement means is arranged at a proximal end of the syringe body and/or protrudes transverse to the longitudinal direction (L) from the syringe body , optionally less than 8 mm, more optionally 6 mm, from the remaining syringe body.

Embodiment eleven is the syringe of any of embodiments one to ten, wherein the plunger comprises plunger engagement means for engagement with an actuator, preferably wherein the plunger engagement means is arranged at a proximal end of the plunger and/or protrudes transverse to the longitudinal direction (L) from the plunger, optionally less than 7 mm, more optionally 5 mm, from the remaining syringe body.

Embodiment twelve is the syringe of any of embodiments one to eleven, wherein the cannula hub comprises a proximal limiter, optionally a circumferential ring, for limiting insertion of the syringe in the longitudinal direction (L) into a grouping mount and/or a reservoir.

Embodiment thirteen is a use of the syringe of any of embodiments one to twelve, for repeatedly flowing liquid through the syringe, wherein the liquid contains nucleic acid sample material and is, via the cannula , withdrawn from a liquid reservoir and discharged into the reservoir multiple times, such that the nucleic acid sample material is sheared and fragmented.

Embodiment fourteen is an apparatus for fragmenting nucleic acid sample material in liquid by means of a plurality of syringes , each syringe having a syringe body and a syringe plunger moveable relative to the syringe body , the apparatus comprising; a support for directly or indirectly supporting a plurality of reservoirs , optionally a PCR plate, for receipt of liquid, and at least one grouping mount for positioning each of the plurality of syringes above the respective reservoir for fluid coupling to the respective reservoir, wherein the plurality of reservoirs and the plurality of syringes are each arranged in a 2D matrix arrangement, the apparatus further comprising; at least one retainer configured to retain one of the plurality of syringe bodies and the plurality of plungers in the 2D matrix arrangement stationary relative to the support, and at least one actuator configured to reciprocate the other one of the plurality of syringe bodies and the plurality of plungers in the 2D matrix arrangement along a longitudinal direction (L), in which the respective body of each of the plurality of syringes extends.

Embodiment fifteen is an apparatus for fragmenting nucleic acid sample material in liquid by means of a plurality of syringes , each syringe having a cannula , a syringe body and a syringe plunger moveable relative to the syringe body , the apparatus comprising a support for receiving a plurality of reservoirs arranged in a predetermined pitch, the support configured as a common support for the plurality of reservoirs, a removable grouping mount comprising a plurality of mounting means arranged in the predetermined pitch, the grouping mount configured as a common mount for the plurality of syringes in the predetermined pitch, each mounting means configured to capture a lower part of the respective syringe and having a through-hole for passing through of at least a respective cannula , the cannula protruding downwards from the lower part of the syringe, the apparatus further comprising, at least one retainer configured to retain one of the plurality of syringe bodies and the plurality of plungers in the predetermined pitch stationary relative to the support, and at least one actuator configured to reciprocate the other one of the plurality of syringe bodies and the plurality of plungers in predetermined pitch along a longitudinal direction (L), in which the respective body of each of the plurality of syringes extends.

Embodiment sixteen is the apparatus of embodiment fourteen, wherein the support is further configured for receiving the plurality of reservoirs arranged in a predetermined pitch, the support configured as a common support for the plurality of reservoirs, the apparatus further comprising, the removable grouping mount comprising a plurality of mounting means arranged in the predetermined pitch, the grouping mount configured as a common mount for the plurality of syringes in the predetermined pitch, each mounting means configured to capture a lower part of the respective syringe and having a through-hole for passing through of at least a respective cannula , the cannula protruding downwards from the lower part of the syringe.

Embodiment seventeen is the apparatus of embodiment fifteen, the support further for directly or indirectly supporting the plurality of reservoirs , optionally a PCR plate, for receipt of liquid, and the grouping mount for positioning each of the plurality of syringes above the respective reservoir for fluid coupling to the respective reservoir, wherein the plurality of reservoirs and the plurality of syringes are each arranged in a 2D matrix arrangement, the at least one retainer configured to retain one of the plurality of syringe bodies and the plurality of plungers in the 2D matrix arrangement stationary relative to the support, and the at least one actuator configured to reciprocate the other one of the plurality of syringe bodies and the plurality of plungers in the 2D matrix arrangement along a longitudinal direction (L), in which the respective body of each of the plurality of syringes extends.

Embodiment eighteen is the apparatus of any of embodiments fifteen to seventeen, wherein the grouping mount is located above the support and configured such that, when the plurality of reservoirs is received by the support and the plurality of syringes is captured by the grouping mount, each cannula extends into the respective reservoir to establish fluid communication with liquid contained in the respective reservoir.

Embodiment nineteen is the apparatus of any of embodiments fourteen to eighteen, wherein the apparatus is configured to repeatedly switch between a pulled-state of the respective plunger of each syringe in which the respective plunger is at least partially pulled out of the body for receipt of liquid in the body , and a pushed-state of the respective plunger of each syringe in which the respective plunger is at least partially received in the respective body and liquid is pushed out of the body , in the 2D matrix arrangement for repeated withdrawal and discharge, respectively, of liquid by means of the reciprocating movement of the actuator along the longitudinal direction (L).

Embodiment twenty is the apparatus of any of embodiments fourteen to nineteen, wherein the 2D matrix arrangement comprises rows running in first (x) and second (y) directions, each of the first and second directions angled, optionally perpendicular, to the longitudinal direction (L), wherein the at least one retainer has a plurality of retainer elements that are adjacent to each other in the second direction (y) and accessible by the syringes in first direction (x), and each retaining element is configured to retain, among the plurality of syringes , a subgroup of syringes aligned in the row running in the first direction (x), and/or the at least one actuator has a plurality of actuator elements that are adjacent to each other in the second direction (y) and accessible by the syringes in the first direction (x), and each actuator element is configured to retain, among the plurality of syringes , a subgroup of syringes aligned in the row running in the first direction (x).

Embodiment twenty-one is the apparatus of any of embodiments fourteen to twenty, wherein the support includes a drawer moveable in a transverse direction (x, y) orthogonal to the longitudinal direction (L).

Embodiment twenty-two is the apparatus of any of any of embodiments fourteen to twenty-one, further comprising a magnetic connector at the grouping mount for detachably attaching the at least one liquid reservoir, optionally at the plurality of reservoirs , more optionally at the well plate, to the grouping mount.

Embodiment twenty-three is the apparatus of any of embodiments fourteen to twenty-two, further comprising a door for access to the support, optionally the door having reinforcement means for reinforcement of the at least one retainer, when the door is in a closed state.

Embodiment twenty-four is the apparatus of any of embodiments fourteen to twenty-three, further comprising at least one pillar extending in the longitudinal direction (L), optionally two pillars running in parallel in the longitudinal direction (L), and supporting the grouping mount and/or the support, optionally at opposite sides in the transverse direction.

Embodiment twenty-five is the apparatus of any of embodiments fourteen to twenty-four, further comprising at least one motor, optionally configured to run at different speeds, more optionally two motors and/or located at a lower part of the apparatus.

Embodiment twenty-six is the apparatus of any of embodiments fourteen to twenty-five, wherein the retainer is configured to restrict movement in the longitudinal direction (L) of the plurality of syringe bodies , more optionally when aligned in the transverse direction (x, y), by abutment of body engagement means of each syringe body against the retainer.

Embodiment twenty-seven is the apparatus of any of embodiments fourteen to twenty-six wherein the actuator is configured to engage with a plunger end of the plunger of each of the plurality of syringes , optionally when aligned in the transverse direction (x, y), by capturing plunger engagement means of each of the plungers .

Embodiment twenty-eight is the apparatus of any of embodiments fourteen to twenty-seven, wherein the retainer is located below the actuator, seen in the longitudinal direction (L), and/or the retainer and the actuator each extend at least in a transverse direction (x, y) perpendicular to the longitudinal direction (L).

Embodiment twenty-nine is a use of the apparatus of any of embodiments fourteen to twenty-eight, wherein the use comprises; arranging the plurality of reservoirs at the grouping mount, arranging the grouping mount comprising the plurality of syringes, each syringe captured by the respective grouping means , in the apparatus, optionally at the support, wherein the grouping mount is located above the support and each cannula extends into the respective reservoir and establishing fluid communication, via the respective cannula , between each syringe and the liquid contained in the respective reservoir.

Detailed embodiments and further advantages and features related to the present disclosure are described in the following with reference to the drawings, wherein these examples or exemplary embodiments shall not be regarded as limiting the subject matter as claimed.

### BRIEF DECSRIPTION OF THE DRAWINGS

Fig. 1 schematically shows in Fig. 1(a) an exploded view of a disassembled syringe according to the disclosure; in Fig. 1(b) a cross-sectional view of a lower part of a syringe according to the disclosure: and in Fig. 1(c) a perspective view of an insert of a syringe according to the disclosure.
Fig. 2 schematically shows in Fig. 2(a) a perspective view of a plurality of syringes in a holder of an apparatus of the disclosure; and in Fig. 2(b) a perspective view of another plurality of syringes in a holder of an apparatus of the disclosure.
Fig. 3 schematically shows in Fig. 3(a) and 3(b) a perspective view of an apparatus of the disclosure, wherein syringes according to the disclosure are received.
Fig. 4 schematically shows a perspective view of an apparatus of the disclosure in which syringes of the disclosure are being placed.
Fig. 5 schematically shows in Fig. 5(a) a perspective view of an apparatus of the disclosure, and in Fig. 5(b) a perspective view of an apparatus of the disclosure, wherein the housing is not shown; and in Fig. 5(c) a perspective view of an apparatus of the disclosure, wherein the door is opened.

### DETAILED DESCRIPTION

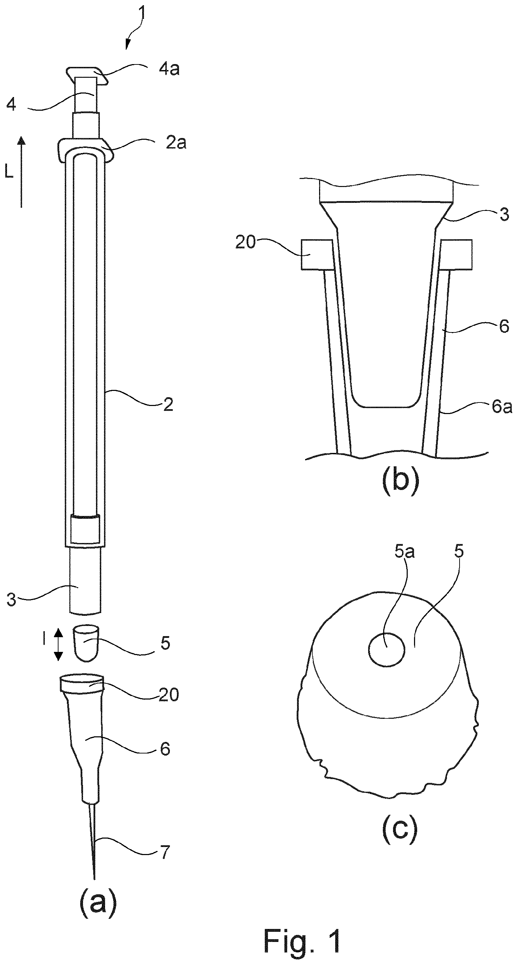

Fig. 1 schematically shows in Fig. 1(a) a perspective view of a disassembled syringe 1 according to the disclosure, in Fig. 1(b) a cross-sectional view of a part of a syringe 1 according to the disclosure, and in Fig. 1(c) a perspective view of an insert 5 of a syringe 1 according to the disclosure.

The syringe 1 is configured for fragmenting nucleic acid sample material in liquid. The syringe 1 comprises a syringe body 2 extending at least in part in a longitudinal direction L of the syringe 1. The syringe body to may have a substantially cylindrical form, the axis of which extends in the longitudinal direction L. Proximal and distal ends may be, in connection with the syringe 1, at an end towards the longitudinal direction L and at an end opposite to/facing away from the longitudinal direction L, respectively. In use, the longitudinal direction L may run vertically.

The syringe 1 further comprises a syringe plunger 4 moveable in the longitudinal direction L and substantially inside the syringe body 2. The syringe plunger 4 is movable between a pulled-state, in which the plunger 4 is at least partially pulled out of the body 2 for receipt of liquid in the body 2, and a pushed-state (shown in Fig. 1(a)) in which the plunger 4 is almost completely received in the body 2 and liquid is pushed out of the body 2.

The syringe 1 further comprises a cannula hub 6 comprising a cannula 7 for repeated withdrawal and discharge of liquid, based on repeated movement of the syringe plunger 4 between the pulled-state and the pushed-state, respectively. More specifically, liquid is withdrawn from a reservoir (not shown in Fig. 1) when the plunger 4 is moved from the pushed state to the pulled state, and liquid is discharged or ejected from the syringe 1, when the plunger 4 is moved from the pulled state to the pushed state. Accordingly, liquid is repeatedly flown through the syringe 1, wherein the liquid contains nucleic acid sample material and is, via the cannula 7, withdrawn from a liquid reservoir (not shown in Fig. 1) and discharged into the reservoir multiple times, such that the nucleic acid sample material is sheared and fragmented.

The syringe 1 further comprises an integrally or separately formed narrowing body 5 comprising a through hole 5a extending in the longitudinal direction L, as shown in Fig 1(c). The through hole 5a fluidly couples the syringe body 2 and the cannula 7, and narrows and/or defines a flow cross-section of the flow of liquid at least in parts between the syringe body 2 and the cannula 7. More specifically, the cross-section of the flow path in the longitudinal direction L is decreased by means of the narrowing body 5, at least in parts along the longitudinal direction L.

As shown in Fig. 1(a) and (c), the narrowing body 5 is embodied as an insert that is insertable into the cannula hub 6 such that a dead space within the cannula hub 6 is at least partly filled by the insert 5. The dead space is defined by one or more interior walls 6a of the cannula hub 6, see Fig. 1(b). Alternatively, the narrowing body 5 may be integrally formed with the cannula hub 6. It is noted that apart from the narrowing body 5 being either integrally formed with the cannula hub 6 or being formed as separate part, all features, functions and advantages of the narrowing body equally apply to both configurations or designs of the narrowing body.

The through hole 5a of the narrowing body 5 has a (e.g. constant along the length of the narrowing body, i.e. in the longitudinal direction L) through-hole diameter and may define a cylindrical flow path. The cannula 7 has an (e.g. constant along the length of the inner cannula, i.e. in the longitudinal direction L) inner cannula diameter, wherein the through-hole diameter is larger than the inner cannula diameter. More specifically, the through-hole diameter may be larger than the inner cannula diameter. Specifically, the through-hole diameter may be about 1.5 to 20 times larger, optionally 1.5 to 10 time larger, still more optionally 2 to 6 times larger than the inner cannula diameter.

The height/length I of the insert 5, seen in the longitudinal direction L, may be between 2.5 mm to 2.8 mm, see Fig. 1(a). This length I corresponds to the length of the through hole 5a.

In one working embodiment as shown in the Figures, the inner diameter of the cannula is 0.2 mm, and the diameter of the through hole of the narrowing body is 1.0 mm. The cannula hub 6 protrudes from the syringe body 2 by about 8.7 mm in the longitudinal direction L of the syringe body 2, wherein the length I of the insert 5 is about 2.65 mm in the longitudinal direction L.

The outer shape of the insert 5 is cone-shaped, as shown in Fig. 1(c). The insert 5 may at least in parts be made of a bioplastic, such as Polylactic acid, and/or is 3D printed. Other manufacturing methods are possible, however.

The cannula 7 may have a blunt distal termination, or may have a sharp tip (e.g. may be a needle), as shown in Fig. 1(a). The needle/cannula 7 protrudes at least 3 mm, optionally at least 5 mm from the cannula hub 6, and/or less than 38 mm (1.5 inch), optionally 26 mm (1.0 inch), more optionally less than 13 mm (0.5 inch), from the cannula hub 6, in the longitudinal direction L.

The syringe 1 further comprises a syringe tip 3 at a distal end of the syringe body 2, when seen in the longitudinal direction L. The syringe tip 3 and the cannula hub 6 are configured such that they are connectable and/or connected to each other via a Luer connection or a luer-lockconnection. Fig. 1(b) shows a connection between the syringe tip 3 and the cannula hub 6 by means of a Luer connection that is not threaded, such as a luer-slip or slip-tip connection.

The cannula 7 is free of a filter defining multiple through-passages. Alternatively, the cannula 7 may comprise a filter for further facilitating the shearing action.

A cannula size is between 18 G and 34 G, optionally 20 G and 32 G, more optionally 25 G to 30 G, still more optionally at 27 G.

As shown in Fig. 1(a) and (b), a distal end of the cannula hub 6 (seen opposite to the longitudinal direction L) tapers, and is configured to engage a liquid reservoir and/or configured to be received by a holder (not shown in Fig. 1).

The syringe body 2 comprises body engagement means 2a for engagement with a retainer (not shown in Fig. 1). The body engagement means 2a are located at the proximal end of the syringe 1 and protrude transverse to the longitudinal direction L from the syringe body 2. The body engagement means 2a may be seen as a circumferential rim around the syringe body 2. The transverse direction may be in the x-y plane in the drawings.

The plunger 4 comprises plunger engagement means 4a for engagement with an actuator (not shown in Fig. 1). The plunger engagement means 4a are arranged at a proximal end of the plunger 4 and protrude transverse to the longitudinal direction L from the plunger 4. The plunger engagement means 4a may be seen as a circumferential rim around the plunger 4.

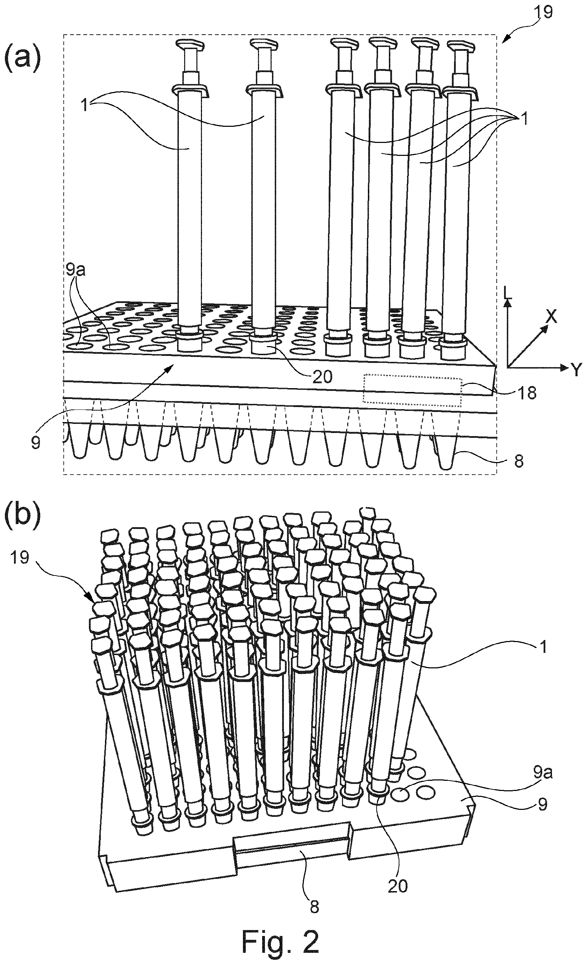

The cannula hub 6 comprises a proximal limiter 20, as shown in Fig. 1(a) and (b). The limiter 20 is formed as a circumferential ring or rim, at the proximal end of the cannula hub. It serves for limiting insertion of the syringe 1 in the longitudinal direction L into a holder 9, as indicated in Fig. 2(a). The outer diameter of such rim may be at least 5 mm and/or less than 9 mm, optionally about 6 mm.

Fig. 2 schematically shows in Fig. 2(a) a perspective view of a plurality of syringes 1 in a holder 9 of an apparatus of the disclosure; and in Fig. 2(b) a perspective view of another plurality of syringes 1 in a holder 9 of an apparatus of the disclosure. The directions x and y may run horizontally, perpendicular to the longitudinal direction L and relative to each other.

A plurality of reservoirs 8 is provided as a 96 well plate. The well plate defines a two-dimensional matrix of 12 x 8 reservoirs, namely 12 reservoirs in the y-direction, and 8 reservoirs in the x-direction. The reservoirs 8 have the same distance/spacing relative to an adjacent reservoir 8, so that the reservoirs are arranged in the same pitch.

The holder 9 is configured as a grouping mount so as to serve as a mount for a grouping of syringes 1 and has a plurality of mounting means 9a formed as through holes. The holder 9 is common to the plurality of syringes 1, so that the plurality (optionally all) of the syringes share a (optionally single) holder 9. Each mounting means 9a is configured for receipt of a syringe 1 and connects to a reservoir 8. The holder 9 is configured such that the plurality of syringes 1 is captured by the grouping mount 9, and each cannula 7 extends into the respective reservoir 8 to establish fluid communication with liquid contained in the respective reservoir 8. The holder 9 is located above the well plate 8, and the syringes 1 are held by the holder 9 such that the syringes 1 stand upright (in the longitudinal direction L) and extend into the reservoirs 8 underneath the holder 9.

The cannula hub 6 of each syringe 1 comprises the proximal limiter 20 limiting insertion of the syringe 1 in the longitudinal direction L into the holder 9 and, as such, into the reservoir 8 below the holder 9, as shown in Fig. 2(a).

In the holder 9 and above the well plate 8, the plurality of syringes 1 forms a grouping 19 of syringes. As the mounting means 9a and the reservoirs 8 are aligned relative to each other and located at predetermined locations, also the grouping 19 of syringes 1 is located at predetermined locations, as shown in Fig. 2(b).

A magnetic connector 18 is provided at the holder 9 for detachably attaching the well plate/reservoirs 8 to the holder 9, see Fig. 2(a).

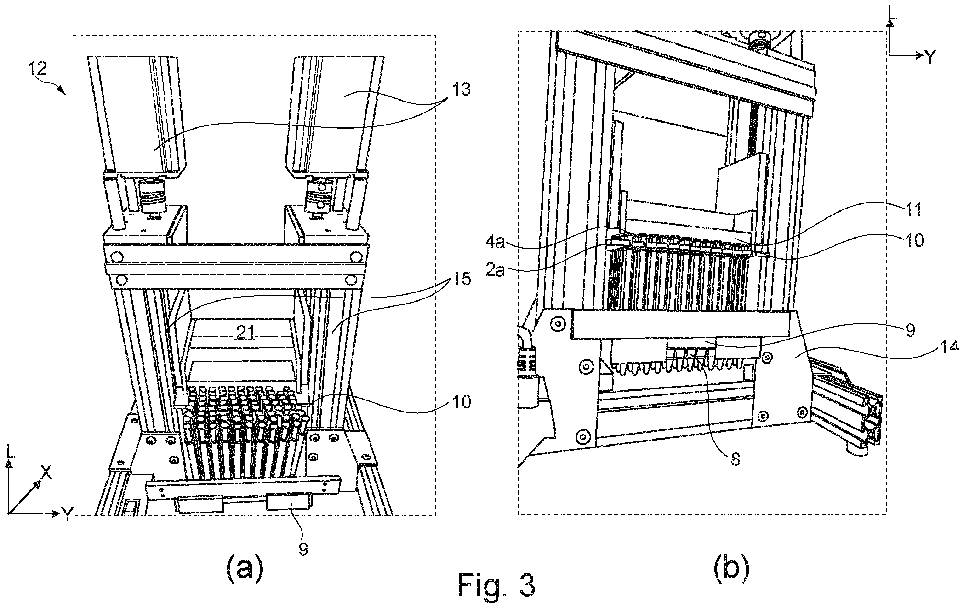

Fig. 3 schematically shows in Fig. 3(a) and 3(b) a perspective view of an apparatus 12 of the disclosure, wherein syringes 1 according to the disclosure are received.

The apparatus 12 is configured for fragmenting nucleic acid sample material in liquid by means of a plurality of syringes 1 of the disclosure. The apparatus 12 comprises a support 14 for directly or indirectly supporting the plurality of reservoirs 8 in the grouping mount 9 for positioning each of the plurality of syringes 1 above the respective reservoir 8 for fluid coupling to the respective reservoir 8.

The plurality of reservoirs 8 and the plurality of syringes 1 are each arranged in a 2D matrix arrangement, and so are the mounting means 9a of the holder 9.

The apparatus 12 further comprises the retainer 10 configured to retain the plurality of syringe bodies 2a in the 2D matrix arrangement stationary relative to the support 14, as shown in Fig. 3(a) and (b). The actuator 11 is configured to reciprocate the plurality of plungers 4 in the 2D matrix arrangement along a longitudinal direction L, in which the respective body 2 of each of the plurality of syringes 1 extends.

Alternatively and not shown, the plurality of plungers 4 may be retained in the 2D matrix arrangement stationary relative to the support 14 and the actuator 11 is configured to reciprocate the syringe bodies 2a in the 2D matrix arrangement along a longitudinal direction L.

The support 14 includes a drawer moveable in a transverse direction x, y orthogonal to the longitudinal direction L. The support 14 is configured for receiving the plurality of reservoirs 8 arranged in a predetermined pitch. The support 14 is configured as a common support for the plurality of reservoirs 8.

The grouping mount 9 comprising a plurality of mounting means 9a arranged in the predetermined pitch. The grouping mount 9 is configured as a common mount for the plurality of syringes in the predetermined pitch, and each mounting means 9a is configured to capture a lower part of the respective syringe 1.

The holder 9 is supported/held by the support 14. When the plurality of reservoirs 8 is received by the support 14 and the plurality of syringes 1 is captured by the holder 9, each cannula 7 extends into the respective reservoir 8 to establish fluid communication with liquid contained in the respective reservoir 8.

The plurality of reservoirs 8 are arranged at the support 14, and the grouping mount 9 comprising the plurality of syringes 1 is arranged in the apparatus 12. The grouping mount 9 is located above the support 14 and each cannula 7 extends into the respective reservoir 8. Accordingly, a fluid communication is established, via the respective cannula 7, between each syringe 1 and the liquid contained in the respective reservoir 8.

The apparatus 12 further comprises two pillars 15 extending parallel to each other and in the longitudinal direction L. The pillars 15 hold the holder 9 and the support 14, at opposite sides in the transverse direction T. The pillars 15 may be regarded as a beam and/or a part of a frame of the apparatus 12.

The apparatus 12 further comprises two motors 13 configured to each run at different speeds. The motors 13 are located at a lower part of the apparatus 12, or an upper part, e.g. above the corresponding pillar 15, as shown in Fig. 3(a). The motors 13 are configured to initiate the reciprocating motion back and forth in the L-direction.

The retainer 10 extends perpendicular to the length direction L and is configured to restrict movement in the longitudinal direction L of the plurality of syringe bodies 2. More specifically, by abutment of body engagement means 2a of each syringe body 2 against the retainer 1 (from below), the syringes 1 are retained and, thus, held stationary.

The actuator 11 is configured to engage with the plunger engagement means 4a of the plunger 4 of each of the plurality of syringes 1. More specifically, the plunger engagement means 4a of each of the plungers 4 are captured (by abutment from below) and pulled upwards, e.g.in the longitudinal direction L. Accordingly, the plungers 4 are reciprocated in the longitudinal direction L.

The apparatus 12 is configured to repeatedly switch between a pulled-state of the respective plunger 4 of each syringe 1 in which the respective plunger 4 is at least partially pulled out of the body 2 for receipt of liquid in the body 2, and a pushed-state of the respective plunger 4 of each syringe 1 in which the respective plunger 4 is at least partially received in the respective body 2 and liquid is pushed out of the body 2, in the 2D matrix arrangement for repeated withdrawal and discharge, respectively, of liquid by means of the reciprocating movement of the actuator 11 along the longitudinal direction L. The 2D matrix arrangement comprises rows running in first x- and second y-directions, wherein each of the first and second directions is perpendicular to the longitudinal direction L.

For example, cycles of about 5 to 500, preferably 50 to 250, more preferably at least 200, e.g. between 200 to 300, up and down movements (reciprocations) of the plungers 4 may be performed. In a working example, each cannula 7 has a size of 27 G and 0.5 inch, and the diameter of the through hole 5a of the narrowing body 5 of the syringe 1 is about 1 mm, and about 200 cycles are run.

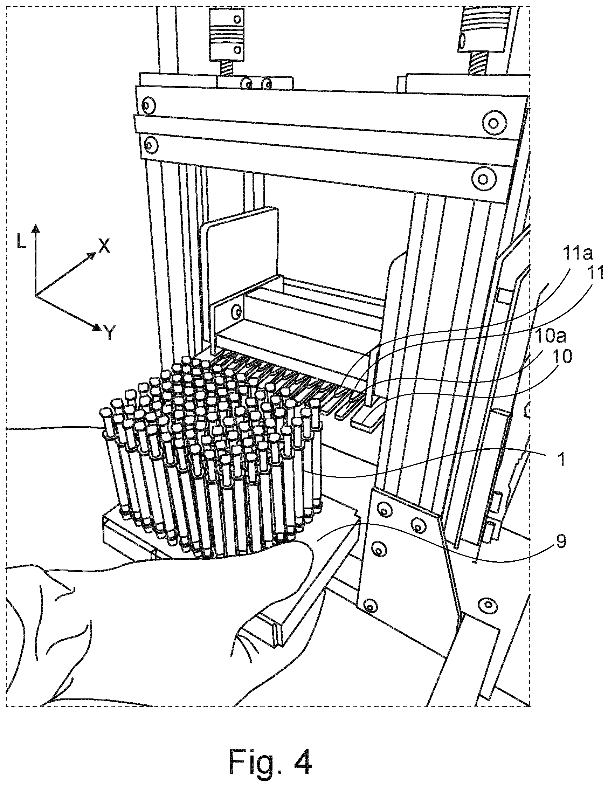

Fig. 4 schematically shows a perspective view of an apparatus 12 of the disclosure in which syringes 1 of the disclosure are being placed. Fig. 4 also shows the retainer 10 and the actuator 11 in detail. The retainer 10 has a plurality of retainer elements 10a that are adjacent to each other in the second direction y and accessible by the syringes 1 in first direction x, and each retaining element 10a is configured to retain, among the plurality of syringes 1, a subgroup of syringes 1 aligned in the row running in the first direction x. The actuator 11 has a plurality of actuator elements 11a that are adjacent to each other in the second direction y and accessible by the syringes 1 in the first direction x, and each actuator element 11a is configured to retain, among the plurality of syringes 1, a subgroup of syringes aligned in the row running in the first direction x.

The retainer 10 is located below the actuator 11, seen in the longitudinal direction L. The retainer 10 and the actuator 11 each extend in the transverse directions x, y perpendicular to the longitudinal direction L. The elements 10a, 11a are configured as bars running in the x-direction and, thus, for engagement with one or two rows of syringes 1 in the x-direction.

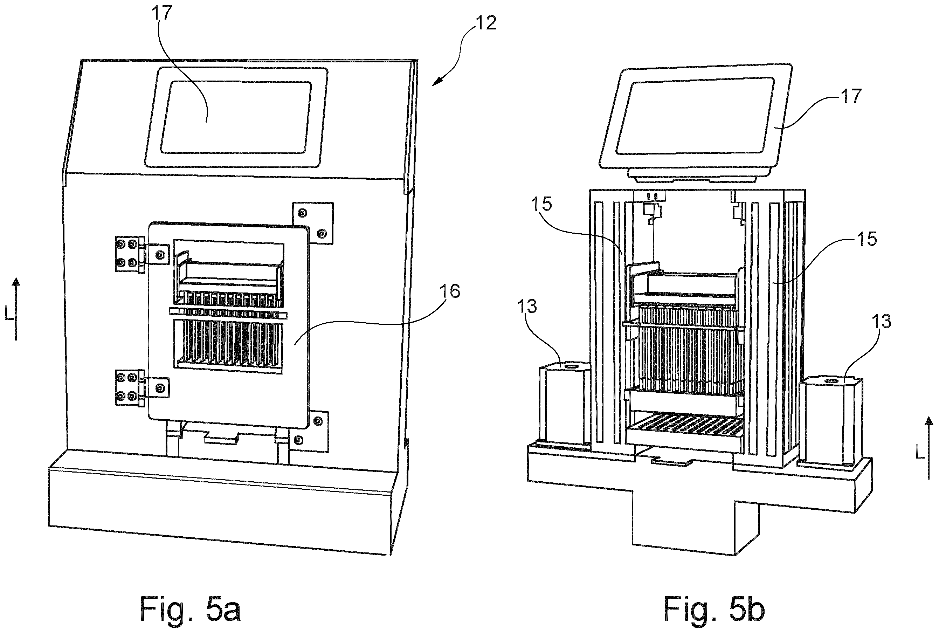

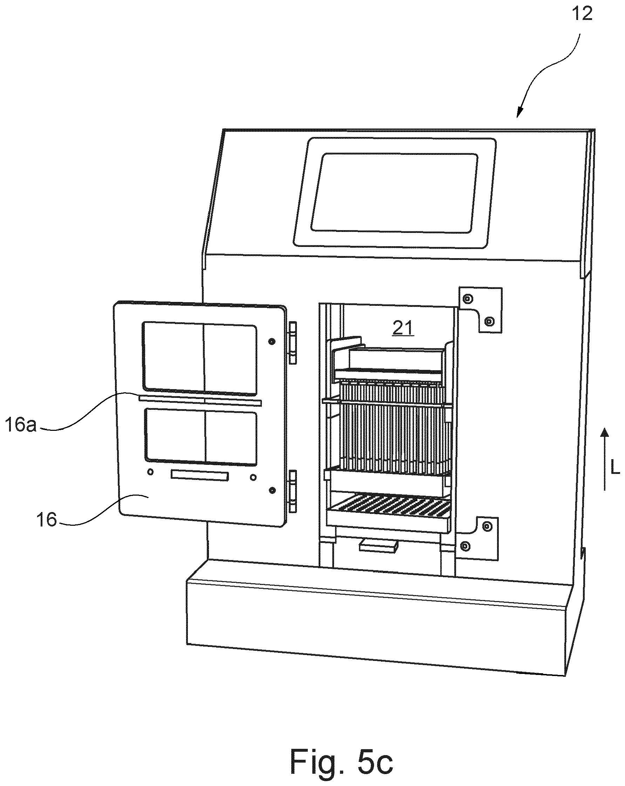

Fig. 5 schematically shows in Fig. 5(a) a perspective view of the apparatus 12 of the disclosure, and in Fig. 5(b) a perspective view of the apparatus 12 of the disclosure, wherein a housing 12 of the apparatus 21 is not shown; an in Fig. 5(c) a perspective view of the apparatus 12 of the disclosure, wherein a door 16 is opened.

The door 16 allows for access to the support 14. The door 16 has reinforcement means 16a (shown in Fig. 5(c)). These serve for reinforcement of the at least one retainer 11, when the door 16 is in a closed state, as shown in Fig. 5(a). The reinforcement means 16 may be formed as a crossbeam or arm, running perpendicular to the longitudinal direction L.

In the embodiment shown in Fig. 5(b), the motors 13 are located at a lower position, next to the basis of the pillars 15.

For the purpose of the present description and of the appended claims, except where otherwise indicated, all numbers expressing amounts, quantities, percentages, sizes and so forth, are to be understood as being modified in all instances by the term "about". Also, all ranges include the maximum and minimum points disclosed and include any intermediate ranges therein, which may or may not be specifically enumerated herein. In this context, therefore, a number A is understood as A ± 10% of A. Within this context, a number A may be considered to include numerical values that are within general standard error for the measurement of the property that the number A modifies. The number A, in some instances as used in the appended claims, may deviate by the percentages enumerated above provided that the amount by which A deviates does not materially affect the basic and novel characteristic(s) of the claimed invention. Also, all ranges include the maximum and minimum points disclosed and include any intermediate ranges therein, which may or may not be specifically enumerated herein.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The mere fact that certain measures are recited in mutually different dependent claims does not indicate that a combination of these measures cannot be used advantageously.

Any reference signs in the claims should not be construed as limiting the scope.

### List of reference signs

1 syringe
2 syringe body
2a body engagement means
3 syringe tip
4 plunger
4a plunger engagement means
5 insert/ narrowing body
5a through hole
6 cannula hub
6a interior wall
7 cannula
8 reservoir / 96 well plate
9 holder/ grouping mount
9a positioning/mounting means
10 retainer
10a retainer element
11 actuator
11a actuator element
12 apparatus
13 motor/drive
14 support
15 pillar
16 door
16a reinforcement means display
17 magnetic connector
18 grouping
19 limiter
20 cavity
21 housing
L longitudinal direction
I length of narrowing body
x first direction
y second direction

## Claims

1. Syringe (1) for fragmenting nucleic acid sample material in liquid, the syringe (1) comprising:
a syringe body (2) extending at least in part in a longitudinal direction (L) of the syringe,
a syringe plunger (4) moveable in the longitudinal direction (L) between a pulled-state, in which the plunger (4) is at least partially pulled out of the body (2) for receipt of liquid in the body (2), and a pushed-state in which the plunger (4) is at least partially received in the body (2) and liquid is pushed out of the body (2), and
a cannula hub (6) comprising a cannula (7) for repeated withdrawal and discharge of liquid, based on repeated movement of the syringe plunger (4) between the pulled-state and the pushed-state, respectively, and an integrally or separately formed narrowing body (5) comprising a through hole (5a) extending in the longitudinal direction (L) and fluidly coupling the syringe body (2) and the cannula (7), and defining a cross-section of the flow of liquid at least in parts between the syringe body (2) and the cannula (7).

2. Syringe of claim 1, wherein the syringe (1) includes an insert (5) as narrowing body insertable into the cannula hub (6) such that a dead space within the cannula hub (6) is at least partly filled by the insert (5), the dead space defined by one or more interior walls (6a) of the cannula hub (6).

3. Syringe of claim 1 or 2, wherein the through hole (5a) has a through-hole diameter and the cannula (7) has an inner cannula diameter, wherein the through-hole diameter is larger than the inner cannula diameter and/or the through-hole diameter is about 1.5 to 20, more optionally 1.5 to 10, still more optionally 2 to 6, times the inner cannula diameter.

4. Syringe of any of the preceding claims 1 to 3, wherein the cannula (7) protrudes at least 3 mm, optionally at least 5 mm from the cannula hub (6), and/or less than 38, optionally less 26 mm, more optionally less than 13 mm, from the cannula hub (6).

5. Syringe of any of the preceding claims 1 to 4, wherein the cannula is free of a filter defining multiple through-passages.

6. Syringe of any of the preceding claims 1 to 5, wherein the narrowing body (5) is at least in part made of a solid material, such as a bioplastic, such as Polylactic acid, and/or is at least in part cone-shaped and/or is 3D printed.

7. Syringe of any of the preceding claims 1 to 6, wherein the syringe (1) further comprises a syringe tip (3) at a distal end of the syringe body (2), wherein the syringe tip (3) and the cannula hub (6) are configured such that they are connectable and/or connected to each other via a Luer connection or a luer-lock connection, and/or a length (l) of the narrowing body in the longitudinal direction (L) is about between 25 and 35%, more optionally between 27 and 33% of the length of the cannula hub (6) in the longitudinal direction (L).

8. Syringe of any of the preceding claims 1 to 7, wherein a distal end of the cannula hub (6) is tapered, optionally configured to engage a liquid reservoir and/or configured to be received by a grouping mount (5).

9. Syringe of any of the preceding claims 1 to 8, wherein a cannula size is between 18 to 34 G, optionally 20 and 32 G, more optionally 25 to 30 G, still more optionally at 27 G.

10. Syringe of any of the preceding claims 1 to 9, wherein the syringe body (2) comprises body engagement means (2a) for engagement with a retainer (10), preferably wherein the body engagement means (2a) is arranged at a proximal end of the syringe body (2) and/or protrudes transverse to the longitudinal direction (L) from the syringe body (2), optionally less than 8 mm, more optionally 6 mm, from the remaining syringe body.

11. Syringe of any of the preceding claims 1 to 10, wherein the plunger (4) comprises plunger engagement means (4a) for engagement with an actuator (11), preferably wherein the plunger engagement means (4a) is arranged at a proximal end of the plunger (4) and/or protrudes transverse to the longitudinal direction (L) from the plunger (4), optionally less than 7 mm, more optionally 5 mm, from the remaining syringe body.

12. Syringe of any of the preceding claims 1 to 11, wherein the cannula hub (6) comprises a proximal limiter (20), optionally a circumferential ring, for limiting insertion of the syringe (1) in the longitudinal direction (L) into a grouping mount (9) and/or a reservoir (8).

13. Use of the syringe (1) of any of the preceding claims 1 to 12, for repeatedly flowing liquid through the syringe, wherein the liquid contains nucleic acid sample material and is, via the cannula (7), withdrawn from a liquid reservoir and discharged into the reservoir multiple times, such that the nucleic acid sample material is sheared and fragmented.

14. Apparatus (12) for fragmenting nucleic acid sample material in liquid by means of a plurality of syringes (1), each syringe (1) having a syringe body (2) and a syringe plunger (4) moveable relative to the syringe body (2),
the apparatus comprising
a support (14) for directly or indirectly supporting
a plurality of reservoirs (8), optionally a PCR plate, for receipt of liquid, and
at least one grouping mount (9) for positioning each of the plurality of syringes (1) above the respective reservoir (8) for fluid coupling to the respective reservoir (8),
wherein the plurality of reservoirs (8) and the plurality of syringes (1) are each arranged in a 2D matrix arrangement,
the apparatus further comprising
at least one retainer (10) configured to retain one of the plurality of syringe bodies (2a) and the plurality of plungers (4) in the 2D matrix arrangement stationary relative to the support (14), and
at least one actuator (11) configured to reciprocate the other one of the plurality of syringe bodies (2a) and the plurality of plungers (4) in the 2D matrix arrangement along a longitudinal direction (L), in which the respective body (2) of each of the plurality of syringes (1) extends.

15. Apparatus of claim 14, wherein the support (14) is further configured for receiving the plurality of reservoirs (8) arranged in a predetermined pitch, the support configured as a common support for the plurality of reservoirs,
the apparatus further comprising
the removable grouping mount (9) comprising a plurality of mounting means (9a) arranged in the predetermined pitch, the grouping mount (9) configured as a common mount for the plurality of syringes in the predetermined pitch, each mounting means (9a) configured to capture a lower part of the respective syringe (1) and having a through-hole for passing through of at least a respective cannula (7), the cannula protruding downwards from the lower part of the syringe.
